(19) Europäisches Patentamt
European Patent Office
Office européen des brevets

(11) Veröffentlichungsnummer: **0 061 004**
**A1**

(12) # EUROPÄISCHE PATENTANMELDUNG

(21) Anmeldenummer: **82101333.1**

(22) Anmeldetag: **12.08.80**

(51) Int. Cl.³: **C 07 C 47/55**
C 07 C 45/29, C 07 C 45/63
C 07 C 43/313, C 07 C 41/56
C 07 D 317/16

(30) Priorität: **22.08.79 DE 2933979**

(43) Veröffentlichungstag der Anmeldung:
**29.09.82 Patentblatt 82/39**

(84) Benannte Vertragsstaaten:
**BE CH DE FR GB IT LI NL**

(60) Veröffentlichungsnummer der früheren Anmeldung nach Art. 76 EPÜ: **0 024 624**

(71) Anmelder: **BAYER AG**
**Zentralbereich Patente, Marken und Lizenzen**
**D-5090 Leverkusen 1, Bayerwerk(DE)**

(72) Erfinder: **Fuchs, Rainer, Dr.**
**Roeberstrasse 8**
**D-5600 Wuppertal 1(DE)**

(72) Erfinder: **Maurer, Fritz, Dr.**
**Roeberstrasse 8**
**D-5600 Wuppertal 1(DE)**

(72) Erfinder: **Priesnitz, Uwe, Dr.**
**Severinstrasse 58**
**D-5650 Solingen 1(DE)**

(72) Erfinder: **Riebel, Hans-Jochem, Dr.**
**In der Beek 92**
**D-5600 Wuppertal 1(DE)**

(72) Erfinder: **Klauke, Erich, Dr.**
**Eichendorffweg 8**
**D-5063 Odenthal(DE)**

(54) **3-Brom-4-fluorbenzaldehyd und seine Acetale und Verfahren zu deren Herstellung.**

(57) Die Erfindung betrifft 3-Brom-4-fluorbenzaldehyd und seine Acetale, sowie Verfahren zu deren Herstellung.

EP 0 061 004 A1

Croydon Printing Company Ltd.

Teilanmeldung aus Anm.-Nr. 80 104 747.3



BAYER AKTIENGESELLSCHAFT 5090 Leverkusen, Bayerwerk
Zentralbereich
Patente, Marken und Lizenzen Rt/bo
Typ IVb/ZP

# 3-Brom-4-fluorbenzaldehyd und seine Acetale und Verfahren zu deren Herstellung

Die Erfindung betrifft 3-Brom-4-fluorbenzaldehyd und seine Acetale, sowie Verfahren zu deren Herstellung.

Es ist bekannt, daß man 4-Fluor-3-phenoxy-benzaldehyd, ein Zwischenprodukt für pestizid wirksame Pyrethroide, erhält, wenn man 4-Fluor-3-phenoxy-benzylbromid mit Hexamethylentetramin umsetzt und das Produkt dieser Umsetzung mit Säuren erhitzt (vergleiche DE-OS 2 7o9 264). Die Ausbeute ist jedoch bei dieser Synthesemethode, wie auch bei der Herstellung der Ausgangsverbindung aus 4-Fluor-3-phenoxy-toluol und N-Brom-succinimid, unbefriedigend.

4-Fluor-3-phenoxy-benzaldehyd-acetale der Formel I

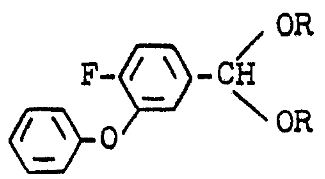

(I)

in welcher die beiden Reste

R einzeln für Alkyl oder zusammen für Alkandiyl (Alkylen) stehen,

werden erhalten, indem man 3-Brom-4-fluor-benzaldehyd-acetale der Formel II

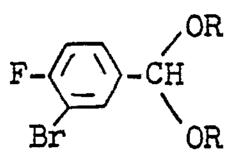

(II)

in welcher

R die oben angegebene Bedeutung hat,

mit Alkali- oder Erdalkali-phenolaten in Gegenwart von Kupfer oder Kupferverbindungen als Katalysatoren und in Gegenwart eines Verdünnungsmittels sowie gegebenenfalls in Gegenwart von Hilfsstoffen aus der Reihe der Alkali- oder Erdalkali-halogenide oder -carbonate und gegebenenfalls in Gegenwart eines basischen Entwässerungsmittels bei Temperaturen zwischen 1oo und 2oo°C umsetzt;

Die neuen Verbindungen der Formel (I) dienen als Zwischenprodukte zur Herstellung von 4-Fluor-3-phenoxy-benzaldehyd durch Umsetzung mit Säuren nach bekannten Acetalspaltungsmethoden.

Die vorliegende Erfindung betrifft:

3-Brom-4-fluor-benzaldehyd der Formel III und seine Acetale der Formel II

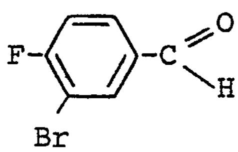 (III) 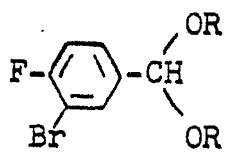 (II)

in welcher die beiden Reste

R einzeln für Alkyl oder zusammen für Alkandiyl (Alkylen) stehen;

ein Verfahren zur Herstellung der neuen Verbindungen der Formel (II), dadurch gekennzeichnet, daß man 3-Brom-4-fluor-benzaldehyd der Formel III

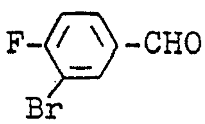 (III)

mit Alkan(di)olen gegebenenfalls in Gegenwart eines Katalysators und/oder eines Wasserbindemittels und gegebenenfalls unter Verwendung eines Verdünnungsmittels bei Temperaturen zwischen 0 und 15o°C umsetzt;

ein Verfahren zur Herstellung von 3-Brom-4-fluor-benzaldehyd, dadurch gekennzeichnet, daß man 3-Brom-4-fluorbenzole der Formel IV

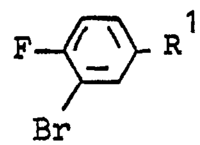

(IV) .

in welcher

$R^1$ für $CH_3$ oder $-CH_2-OH$ steht

in üblicher Weise in den Aldehyd überführt.

Überraschenderweise kann über die vorstehend aufgeführten neuen Zwischenprodukte 4-Fluor-3-phenoxy-benzaldehyd auf einfachere Weise und in besserer Ausbeute als nach dem oben erwähnten bekannten Verfahren hergestellt werden.

Das Verfahren zur Herstellung der neuen 4-Fluor-3-phenoxy-benzaldehyd-acetale kann bei Verwendung von 3-Brom-4-fluor-benzaldehyd-propylenacetal und Kaliumphenolat als Ausgangsverbindungen durch folgendes Reaktionsschema skizziert werden:

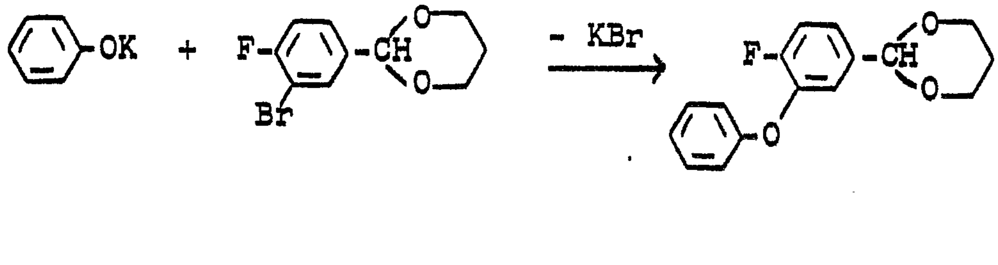

Alkali- oder Erdalkali-phenolate, welche als Ausgangsstoffe verwendet werden können, sind z.B. Natrium-, Kalium- und Magnesium-phenolat. Natriumphenolat wird als Ausgangsverbindung bevorzugt.

Als Katalysatoren werden Kupfer oder Kupferverbindungen verwendet. Als Beispiele hierfür seien Kupfer, Kupfer(I)-oxid, Kupfer(II)oxid, Kupfer(I)chlorid und Kupfer(I)-bromid genannt.

Als Verdünnungsmittel werden vorzugsweise aprotisch polare Solventien verwendet. Beispiele hierfür sind Dimethylformamid, Dimethylacetamid, N-Methylpyrrolidon, Dimethylsulfoxid, Tetramethylensulfon, Hexamethylphosphonsäuretriamid und Bis-(2-methoxyethyl)-ether (Diglyme). Letzteres wird besonders bevorzugt.

Hilfsstoffe aus der Reihe der Alkali- oder Erdalkalihalogenide oder -carbonate sind z.B. Kalium- und Magnesiumcarbonat. Diese Hilfsstoffe werden vorzugsweise dann verwendet, wenn Natriumphenolat als Ausgangsverbindung eingesetzt wird.

Basische Entwässerungsmittel, welche verwendet werden können, sind z.B. Natriumhydroxid, Kaliumhydroxid, Calciumhydroxid, Kaliumcarbonat, Natriumamid, Natriumhydrid und Calciumhydrid.

Die Reaktionstemperatur wird zwischen 100 und 200°C, vorzugsweise zwischen 130 und 170°C gehalten. Das Verfahren wird gewöhnlich unter Normaldruck durchgeführt.

Auf 1 Mol 3-Brom-4-fluor-benzaldehyd-acetal der Formel (II) setzt man 1 bis 1,5 Mol, vorzugsweise 1 bis 1,2 Mol Phenolat, o,ol bis o,5 Mol, vorzugsweise o,1 bis o,5 Mol Kupferkatalysator, loo bis 4oo ml Verdünnungsmittel, gegebenenfalls o,ol bis o,5 Mol eines Hilfsstoffes aus der Reihe der Alkali- oder Erdalkali-halogenide oder -carbonate und gegebenenfalls bis zu o,2 Mol eines Entwässerungsmittels ein.

In einer bevorzugten Ausführungsform wird das Phenolat in einem Verdünnungsmittel, zu dem man gegebenenfalls ein Entwässerungsmittel gegeben hat, vorgelegt, mit dem Kupferkatalysator und gegebenenfalls mit dem Hilfsstoff aus der Reihe der Alkali- oder Erdalkalihalogenide oder -carbonate versetzt und das Gemisch wird auf die Reaktionstemperatur aufgeheizt. Dann wird das 3-Brom-4-fluor-benzaldehyd-acetal eindosiert und das Gemisch bis zum Reaktionsende gerührt. Zur Aufarbeitung, welche nach üblichen Methoden erfolgen kann, verdünnt man beispielsweise mit Toluol, filtriert und destilliert vom Filtrat das Lösungsmittel unter vermindertem Druck ab, wobei das Rohprodukt als Rückstand verbleibt.

Die neuen Verbindungen der Formel (I) können zur Herstellung von 4-Fluor-3-phenoxybenzaldehyd, welcher als Zwischenprodukt für Pyrethroide bekannt ist (vergleiche DE-OS 2 7o9 264), verwendet werden. Die Herstellung von 4-Fluor-3-phenoxy-benzaldehyd durch Acetalspaltung von Verbindungen der Formel (I) mit Säuren kann bei Einsatz

von 4-Fluor-3-phenoxy-benzaldehyd-propylenacetal durch folgendes Formelschema skizziert werden:

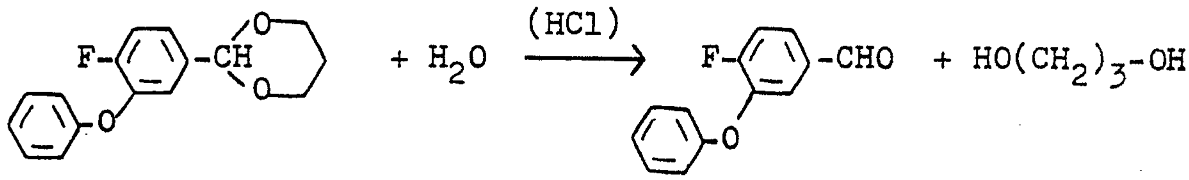


Die Acetalspaltung zur Herstellung von 4-Fluor-3-phenoxy-benzaldehyd kann nach üblichen Methoden durchgeführt werden. In einer bevorzugten Verfahrensweise werden die Verbindungen der Formel (I) in wässrigem Alkohol gelöst und mit der katalytischen Menge einer starken Säure, wie z.B. Salzsäure, bei Raumtemperatur stehen gelassen. Nach einigen Stunden wird das Reaktionsgemisch mit einem mit Wasser nicht mischbaren Lösungsmittel, wie z.B. Toluol, versetzt, die organische Phase abgetrennt, mit Wasser gewaschen, getrocknet, filtriert und destilliert.

Die als Zwischenprodukte zu verwendenden neuen 3-Brom-4-fluor-benzaldehyd-acetale sind durch Formel (II) definiert. Vorzugsweise stehen darin die Reste

R einzeln für $C_1$-$C_4$-Alkyl oder zusammen für $C_2$-$C_5$-Alkandiyl, insbesondere für $-CH_2-CH_2-$.

Das Verfahren zur Herstellung der neuen 3-Brom-4-fluor-benzaldehyd-acetale kann bei Verwendung von Propan-1,3-diol durch folgendes Reaktionsschema skizziert werden:

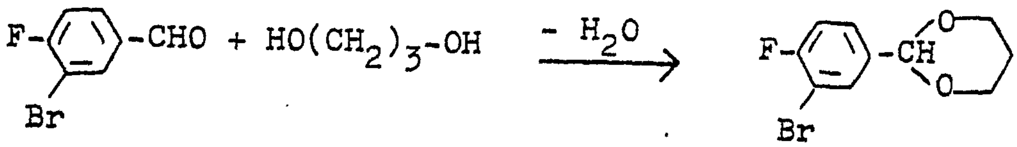

Alkanole bzw. Alkandiole, welche als Acetalisierungsmittel eingesetzt werden können, sind z.B. Methanol, Ethanol, Propanole und Butanole sowie Ethan-1,2-diol (Ethylenglykol) und Propan-1,3-diol. Ethylenglykol wird als Acetalisierungskomponente besonders bevorzugt.

Geeignete Katalysatoren sind z.B. Chlorwasserstoff, Bromwasserstoff, Schwefelsäure, p-Toluolsulfonsäure, Bortrifluorid, Zinkchlorid und saure Ionenaustauscherharze.

Geeignete Wasserbindemittel sind z.B. Orthoameisensäuretriethylester, Dimethylsulfit und Chlortrimethylsilan.

Das Verfahren wird gegebenenfalls in Gegenwart von Verdünnungsmitteln durchgeführt. Als solche sind insbesondere Lösungsmittel geeignet, mit denen Wasser durch azeotrope Destillation aus dem Reaktionsgemisch entfernt werden kann. Als Beispiele seien Benzol, Toluol und Xylol genannt.

Die Reaktionstemperatur liegt zwischen 0 und 150°C. Das Verfahren wird im allgemeinen bei Normaldruck durchgeführt.

Auf 1 Mol 3-Brom-4-fluor-benzaldehyd setzt man 1 bis 1,5 Moläquivalente, vorzugsweise 1 bis 1,2 Moläquivalente Alkan(di)ol und gegebenenfalls 2 bis 3 Mol, vorzugsweise 2 bis 2,5 Mol eines Wasserbindemittels ein.

In einer bevorzugten Ausführungsform werden 3-Brom-4-fluor-benzaldehyd, das Alkan(di)ol und ein Wasserbindemittel vermischt und einige Stunden erhitzt. Zur Aufarbeitung, welche nach üblichen Methoden erfolgen kann, wird beispielsweise mit einem mit Wasser nicht mischbaren Lösungsmittel, wie z.B. Toluol, verdünnt, die Lösung mit Eiswasser gewaschen, getrocknet, filtriert und destilliert.

Das Verfahren zur Herstellung von 3-Brom-4-fluor-benzaldehyd aus 3-Brom-4-fluor-benzylalkohol wird vorzugsweise unter Verwendung von Verdünnungsmitteln durchgeführt. Als solche kommen gegebenenfalls Wasser und/oder aliphatische oder aromatische, gegebenenfalls halogenierte Kohlenwasserstoffe, wie z.B. Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol in Frage.

Die Reaktionstemperatur liegt zwischen 0 und 100°C, vorzugsweise zwischen 10 und 60°C. Das Verfahren wird gewöhnlich bei Normaldruck durchgeführt.

In einer bevorzugten Variante (a) wird als Oxidationsmittel Chrom(VI)oxid-Pyridin-Chlorwasserstoff (1/1/1) verwendet. Zu einer Lösung dieses Oxidationsmittels, beispielsweise in Methylenchlorid, gibt man eine Lösung von 3-Brom-4-fluor-benzyl-alkohol, ebenfalls in Methylenchlorid, und rührt das Gemisch bis zum Reaktionsende. Zur Aufarbeitung wird, gegebenenfalls nach Abdekantieren von ungelösten Komponenten, destilliert.

In einer zweiten bevorzugten Variante (b) wird Salpetersäure als Oxidationsmittel und Wasser als Verdünnungsmittel verwendet. Hierzu gibt man den 3-Brom-4-fluor-benzylalkohol und rührt das Gemisch einige Stunden bei Raumtemperatur. Zur Aufarbeitung wird mit Natronlauge alkalisiert, mit einem mit Wasser nicht mischbaren Lösungsmittel, wie z.B. Toluol, extrahiert, oder Extrakt mit Wasser gewaschen, getrocknet, filtriert und destilliert.

In einer dritten bevorzugten Variante (c) wird Chromsäure bzw. Dichromat und Schwefelsäure als Oxidationsmittel verwendet. Verdünnungsmittel ist hierbei vorzugsweise ein Zweiphasensystem aus Wasser und einem der oben genannten organischen Lösungsmittel. Als Katalysatoren werden vorzugsweise Verbindungen verwendet, welche zum Transfer von Anionen aus Wasser in organische Lösungsmittel geeignet sind. Beispiele hierfür sind Benzyl-triethyl-ammonium-hydrogensulfat, Tetrabutyl-ammonium-bromid und Methyl-tricapryl-ammoniumchlorid (Aliquat 336).

Zur Durchführung der Verfahrensvariante (c) wird 3-Brom-4-fluor-benzylalkohol in einem organischen Lösungsmittel, wie z.B. Methylenchlorid, vorgelegt und dazu werden Schwefelsäure, Wasser, Katalysator und Dichromat gegeben. Das Reaktionsgemisch wird einige Stunden gerührt. Zur Aufarbeitung wird mit Wasser verdünnt, die organische Phase abgetrennt, mit Wasser gewaschen, getrocknet, filtriert und destilliert.

3-Brom-4-fluor-benzylalkohol und ein Verfahren zu dessen Herstellung ist Gegenstand einer noch nicht zum Stand der Technik gehörenden Patentanmeldung (Le A 19 875).
Man erhält diese Verbindung, wenn man 3-Brom-4-fluor-benzoylfluorid mit einem Hydridkomplex, wie z.B. Natriumtetrahydridoborat, gegebenenfalls unter Verwendung eines Verdünnungsmittels, wie z.B. iso-Propanol, bei Temperaturen zwischen 0 und 50°C umsetzt. Das Reaktionsgemisch wird bis zum Ende der Umsetzung gerührt, mit Eiswasser verdünnt und angesäuert. Dann wird mit einem mit Wasser nicht mischbaren Lösungsmittel, wie z.B. mit Methylenchlorid, extrahiert, der Extrakt getrocknet, filtriert und destilliert.

Das als Ausgangsverbindung zu verwendende 3-Brom-4-fluor-benzoylfluorid ist Gegenstand einer ebenfalls noch nicht zum Stand der Technik gehörenden Patentanmeldung (vergleiche P 2 915 738/Le A 19 590).
Man erhält diese Verbindung, wenn man 4-Chlor-benzoylchlorid durch Umsetzung mit Kaliumfluorid in 4-Fluor-benzoylfluorid umwandelt und letzteres zu 3-Brom-4-fluor-benzoylfluorid bromiert gemäß nachstehendem Formelschema:

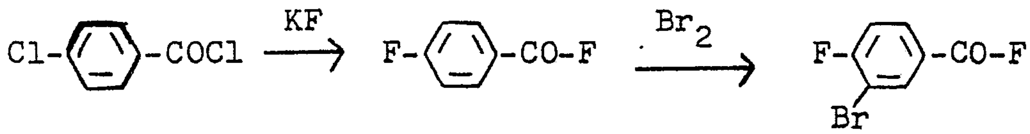


4-Chlor-benzoylchlorid wird mit Kaliumfluorid beispielsweise in Tetramethylensulfon bei Temperaturen zwischen 2oo und 22o°C umgesetzt und das Reaktionsgemisch destillativ aufgearbeitet. Man erhält 4-Fluor-benzoylfluorid vom Siedepunkt 53°C/2o mBar (Brechungsindex: $n_D^{2o}$=1,4792).

4-Fluor-benzoylfluorid wird mit elementarem Brom in Gegenwart von 1% Eisen(III)chlorid bei 7o bis 75°C umgesetzt. Bei einem Ansatz von 1 Mol erhält man nach Destillation 4o g unverändertes Ausgangsmaterial zurück und 182 g eines Gemisches von 3-Brom-4-fluor-benzoylfluorid (Siedepunkt: 82-83°C/15 mBar; Brechungsindex: $n_D^{2o}$ = 1,5315; Schmelzpunkt: 32-34°C) und 3-Brom-4-fluorbenzoylbromid (Siedepunkt: 123°C/15 mBar; Schmelzpunkt: 35-37°C).

Der nach obigem Verfahren herzustellende 3-Brom-4-fluorbenzaldehyd (III) kann prinzipiell auch nach anderen Methoden erhalten werden, beispielsweise durch Bromierung von 4-Fluor-benzaldehyd oder durch Seitenkettenhalogenierung von 3-Brom-4-fluor-toluol und anschließende Sommelet-Reaktion.

Beispiel 1:

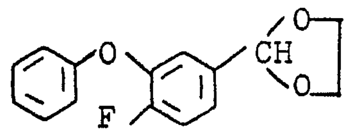

Zu einer Suspension von 3,2 g (27,5 mMol) Natriumphenolat in 3 ml Diglyme gibt man zur Entwässerung o,1 g (3,5 mMol) Natriumhydrid und versetzt die Mischung dann mit o,o5 g Kupfer(I)oxid (o,35mMol) sowie mit o,5 g (6,5 mMol) Kaliumchlorid. Das Reaktionsgemisch wird unter Inertgas (z.B. Argon) auf 155°C erhitzt und bei dieser Temperatur mit 6,2 g (25 mMol) 3-Brom-4-fluor-benzaldehyd-ethylenacetal versetzt. Man rührt 7 Stunden bei 155°C nach, gibt nach Abkühlen auf Raumtemperatur 5o ml Toluol zu und saugt vom anorganischen Material ab. Das Filtrat wird im Vakuum vom Lösungsmittel befreit. Man erhält so 6,o g eines Produktes mit einem Gehalt von 87% von 4-Fluor-3-phenoxy-benzaldehyd-ethylenacetal (Entspricht einer Ausbeute von 8o% der Theorie).

Beispiel 2:

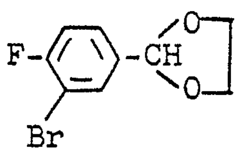

Eine Mischung aus 2o,3 g (o,1 Mol) 3-Brom-4-fluor-benzaldehyd und 6,8 g (o,11 Mol) Ethandiol-1,2 wird mit 26 g (o,24 Mol) Trimethylchlorsilan versetzt und 3 Stunden auf loo°C erhitzt. Nach Abkühlen auf Raumtemperatur gibt man loo ml Toluol zu und schüttelt 2 mal mit je 5o ml Eiswasser. Die organische Phase wird über Natriumsulfat getrocknet und im Vakuum eingedampft. Den Rückstand destilliert man im Vakuum. Man erhält so 21 g (85% der Theorie) 3-Brom-4-fluor-benzaldehydethylenacetal in Form eines farblosen Öles mit dem Siedepunkt 79-81°C/o,1 mm Hg.

Beispiel 3:

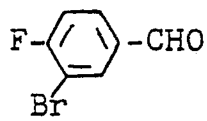

Variante (a)

Eine Lösung von 45,9 g (o,225 Mol) 3-Brom-4-fluor-benzylalkohol in 45 ml Methylenchlorid wird zu einer Suspension von 78 g 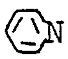 · $CrO_3$ · HCl in 45o ml Methylenchlorid getropft. Dabei steigt die Temperatur des Gemisches bis etwa 4o°C an. Man rührt anschließend 1 Stunde nach, dekantiert die organische Phase von den Chromsalzen und destilliert das Lösungsmittel im Vakuum ab. Der Rückstand wird destilliert. Man erhält so 39 g (86% der Theorie) 3-Brom-4-fluor-benzaldehyd mit dem Siedepunkt 63-65°C/o,3 mm Hg.

Variante (b)

Zu einer Mischung von lo g Salpetersäure (Dichte 1,4) und 5 ml Wasser gibt man bei 3o-35°C lo,2 g (o,o5 Mol) 3-Brom-4-fluor-benzylalkohol und rührt das Gemisch anschließend 5 Stunden bei Raumtemperatur. Nach Zugabe von 3o g Eis versetzt man die Mischung mit Natronlauge, bis ein pH-Wert von 13 erreicht ist und schüttelt dann

mit 200 ml Toluol aus. Die organische Phase wird drei mal mit je 50 ml Wasser gewaschen, über Natriumsulfat getrocknet und dann eingedampft. Der Rückstand wird destilliert. Man erhält so 6,1 g (60 % der Theorie) 3-Brom-4-fluor-benzaldehyd in Form eines langsam erstarrenden Öles mit dem Schmelzpunkt 29 - 31°.

Variante (c)

Zu einer Lösung von 2o,5 g (o,1 Mol) 3-Brom-4-fluor-benzylalkohol in 25o ml Methylenchlorid gibt man bei Raumtemperatur eine Mischung aus 29,4 g (o,3 Mol) Schwefelsäure, 5o ml Wasser und 2 ml Aliquat 336 (Tricaprylmethylammoniumchlorid). Danach versetzt man Reaktionsgemisch mit 9,7 g (o,o33 Mol) Kaliumdichromat und hält die Temperatur während 2 Stunden durch schwaches Kühlen auf ca. 25°C. Nach Zugabe von loo ml Wasser trennt man die organische Phase ab und schüttelt das Wasser noch einmal mit loo ml Methylenchlorid aus. Die organischen Phasen werden 2 mal mit je loo ml Wasser, dann einmal mit loo ml gesättigter Natriumhydrogencarbonatlösung und noch einmal mit loo ml Wasser gewaschen, getrocknet, über Natriumsulfat und im Vakuum eingedampft. Der Rückstand wird destilliert. Man erhält auf diese Weise 16,3 g (81% der Theorie) 3-Brom-4-fluor-benzaldehyd als farbloses Öl mit dem Siedepunkt 65°C/o,3 mm Hg.

Beispiel 4:

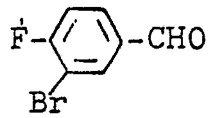

Zu einer Mischung aus 166 g Aluminiumchlorid und 15o ml 1,2-Dichlorethan werden bei einer Innentemperatur von 3o°C 62 g 4-Fluor-benzaldehyd tropfenweise gegeben und das Gemisch wird etwa 3o Minuten nachgerührt. Dann werden 88 g Brom bei einer Innentemperatur zwischen 3o und 4o°C zugetropft. Das Reaktionsgemisch wird etwa 2 Stunden nachgerührt und dann auf Eis gegossen. Nach Abtrennen der organischen Phase wird die wässrige Phase mit 1,2-Dichlorethan nachextrahiert. Die vereinigten organischen Phasen werden mit Wasser gewaschen, mit Natriumsulfat getrocknet und filtriert. Nach Abdestillieren des Lösungsmittels unter vermindertem Druck erhält man als Rückstand ein Rohprodukt, welches nach gaschromatographischer Analyse 78,8% 3-Brom-4-fluor-benzaldehyd und 17,6% 4-Fluor-benzaldehyd enthält. Nach Vakuumdestillation erhält man 58 g 3-Brom-4-fluor-benzaldehyd vom Siedepunkt 1o8°C/25 mBar und vom Brechungsindex $n_D^{20}$: 1,5737. Das Produkt erstarrt allmählich: Schmelzpunkt 3o-31°C.

Beispiel zur Herstellung von 4-Fluor-3-phenoxy-benzaldehyd:

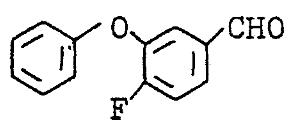

Eine Lösung von 26 g (o,1 Mol) 4-Fluor-3-phenoxy-benzaldehyd-ethylenacetal in 6o ml Ethanol, 2o ml Wasser und

1 ml konzentrierter Salzsäure wird 3 Stunden bei Raumtemperatur aufbewahrt. Dann destilliert man das Ethanol im Vakuum ab und versetzt den Rückstand mit 1oo ml Toluol. Das Wasser wird abgetrennt, die organische Phase zwei mal mit je 5o ml Wasser gewaschen, über Natriumsulfat getrocknet und im Vakuum eingedampft. Den Rückstand destilliert man im Vakuum. Man erhält auf diese Weise 19,6 g (91% der Theorie) 4-Fluor-3-phenoxy-benzaldehyd in Form eines farblosen Öles mit dem Siedepunkt 1o2-1o4$^{o}$C/o,1 mm Hg.

Patentansprüche

1. 3-Brom-4-fluorbenzaldehyd sowie seine Acetale der Formeln II und III

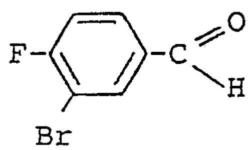

(III)

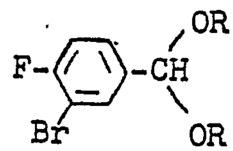

(II)

in welcher die beiden Reste

R einzeln für Alkyl oder zusammen für Alkandiyl (Alkylen) stehen.

2. Verfahren zur Herstellung von 3-Brom-4-fluor-benzaldehyd und seinen Acetalen, dadurch gekennzeichnet, daß man 3-Brom-4-fluor-benzole der Formel

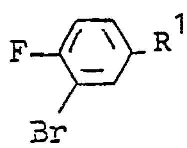

in welcher

$R^1$ für $CH_3$ oder $-CH_2-OH$ steht,

in üblicher Weise in den Aldehyd überführt und diesen gegebenenfalls mit Alkan(di)olen gegebenenfalls in Gegenwart eines Katalysators und/oder eines Wasserbindemittels und gegebenenfalls unter Verwendung eines Verdünnungsmittels bei Temperaturen zwischen 0 und 150°C umsetzt.

Europäisches Patentamt

# EUROPÄISCHER RECHERCHENBERICHT

0061004

Nummer der Anmeldung

EP 82 10 1333

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| E | DE-A-3 026 959 (BAYER)<br>* Beispiele 1,2 *<br>--- | 1 | C 07 C 47/55<br>C 07 C 45/29<br>C 07 C 45/63<br>C 07 C 43/313<br>C 07 C 41/56<br>C 07 D 317/16 |
| E | EP-A-0 024 611 (BAYER)<br>* Beispiel 4 *<br>--- | 1 | |
| E | EP-A-0 034 741 (RIEDEL-DE HAEN)<br>* Ansprüche 1,2,8; Beispiele 2,6 *<br>--- | 1,2 | |

RECHERCHIERTE SACHGEBIETE (Int. Cl. 3)

C 07 C 47/00
C 07 C 43/00
C 01 D 317/00

Der vorliegende Recherchenbericht wurde für alle Patentansprüche erstellt.

| Recherchenort | Abschlußdatum der Recherche | Prüfer |
|---|---|---|
| DEN HAAG | 07-07-1982 | BONNEVALLE E.I.H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82